Europäisches Patentamt

European Patent Office

Office européen des brevets

(11) Publication number: **0 381 027 B1**

# EUROPEAN PATENT SPECIFICATION

(45) Date of publication of patent specification: **07.12.94**

(51) Int. Cl.5: **C12N 15/74**, C12N 15/53, C12P 7/60

(21) Application number: **90101371.4**

(22) Date of filing: **24.01.90**

(54) Shuttle vectors useful among microorganisms belonging to Escherichi coli, Gluconobacter and Acetobacter.

(30) Priority: **02.02.89 EP 89101771**

(43) Date of publication of application:
**08.08.90 Bulletin 90/32**

(45) Publication of the grant of the patent:
**07.12.94 Bulletin 94/49**

(84) Designated Contracting States:
**AT BE CH DE DK FR GB IT LI NL**

(56) References cited:
**EP-A- 0 276 832**
**EP-A- 0 292 303**
**WO-A-89/06688**
**US-A- 4 680 264**

**J. FERMENT. TECHNOL., vol. 63, no. 1, 1985, pages 1-4; T. INOUE et al.:"Efficient introduction of vector plasmids into acetic bacteria"**

**AGRICULTURAL AND BIOLOGICAL CHEMIS-TRY, vol. 49, no. 7, July 1985, pages 2083-2090, Tokyo, JP; M. FUKAYA et al.: "Construction of new shuttle vectors forAcetobacter"**

(73) Proprietor: **F. HOFFMANN-LA ROCHE AG**
**Postfach 3255**
**CH-4002 Basel (CH)**

(72) Inventor: **Fujiwara, Akiko Parkcity**
**Mizonokuchi L-202**
**Hisamoto 30-1, Takatsu-ku**
**Kawasaki-shi, Kanagawa-ken (JP)**
Inventor: **Hoshino, Tatsuo**
**Fueta 808-47**
**Kamakura-shi, Kanagawa-ken (JP)**
Inventor: **Shinjoh, Masako, Dai 3 Ohfuna Park**
**Town G-612**
**Kasama-cho 1555-1, Sakae-Ku**
**Yokohama-shi, Kanagawa-ken (JP)**

(74) Representative: **Mezger, Wolfgang, Dr. et al**
**Grenzacherstrasse 124**
**Postfach 3255**
**CH-4002 Basel (CH)**

FUNDAMENTAL AND APPLIED BIOHYDROMETALLURGY, PROCEEDINGS OF THE SIXTHINTERNATIONAL SYMPOSIUM ON BIOHYDROMETALLURGY, Vancouver, 21st-24th August1985, edited by R.W. LAWRENCE, pages 419-427, Elsevier, Amsterdam, NL; D.E.RAWLINGS et al.: "Characterization of a broad-host-range mobilizableThiobacillus ferrooxidans plasmid and the construction of Thiobacillus cloningvectors"

**Description**

The present invention is concerned with novel recombinant DNA shuttle vectors which are useful as shuttle vectors among microorganisms belonging to Escherichia coli, genus Gluconobacter and genus Acetobacter. The present invention also relates to transconjugants of genus Gluconobacter or genus Acetobacter harboring the shuttle vector DNA or its derivatives. Furthermore, the present invention relates to a process for producing transconjugants of genus Gluconobacter or genus Acetobacter harboring the shuttle vector DNA.

A few systems of gene transfer into Gluconobacter oxydans were reported in previous publications. Murooka et al. (J. Bacteriol. 145, 358-368 [1981]) reported conjugal mating of a strain of Gluconobacter oxydans with a strain of a Escherichia coli harboring RP4::Mu. The frequency of appearance of a transconjugant was $10^{-10}$/recipient. Fukaya et al. (Agric. Biol. Chem. 49, 2407-2411 [1985]) reported transformation of Gluconobacter oxydans with a recombinant plasmid which was constructed from an endogenous plasmid of Gluconobacter oxydans and a plasmid of Escherichia coli. The transformation frequency was very low (about $10^2$ transformants/μg DNA; in other words, about $10^{-9}$ transformants/recipient). Thus, these systems are not satisfactorily efficient for a gene transfer into Gluconobacter oxydans.

Usually, it is required to obtain thousands of transconjugants or transformants in one experiment for a practical use, e.g., for construction of a genomic library.

European Patent Application Publication No. 276 832 (EP 276 832) already discloses a plasmid with two maker genes, a Mob site and a replication origin which is functional in E. coli and Gluconobacter oxydans.

In accordance with the present invention, novel and highly useful shuttle vectors are obtained by combining maker genes to different replication origins, one functional in Escherichia coli and one functional in Gluconobacter oxydans and a Mob site. Such vectors show a higher expression rate and stability of the plasmid in the host cell than plasmids disclosed in EP 276 832.

Thus one aspect of the present invention concerns novel recombinant DNA vectors which comprise two different replication origins, one functional in Escherichia coli and one functional in Gluconobacter oxydans, one or more marker genes and a Mob site. The DNA vector may further comprise one or more inserts selected from the group consisting of DNA sequences having multicloning sites, expression control sequences, cos sites, terminator sequences, ribosome binding sites, DNA sequences encoding signal peptides and/or proteins. Another aspect of the present invention concerns transconjugants of genus Gluconobacter or genus Acetobacter into which the said vectors are introduced. A further aspect of the present invention concerns a process for producing the said transconjugants which comprises contacting a strain of genus Gluconobacter or genus Acetobacter with a strain of Escherichia coli transformed with the said vector, under conjugal mating conditions.

The shuttle vectors of the present invention can be transferred among any strains belonging to Escherichia coli, genus Gluconobacter and genus Acetobacter, and are replicable in any of these strains.

Suitable Escherichia coli hosts for the shuttle vectors of the persent invention are any strains of Escherichia coli used in recombinant DNA technology, e.g., E. coli K-12, E. coli C600, E. coli HB101, E. coli ED8767 or E. coli S17-1. Suitable Gluconobacter hosts for the shuttle vectors of the present invention are any strains belonging to the genus Gluconobacter. According to the newest classification, all the strains belonging to the genus Gluconobacter fall into the species Gluconobacter oxydans (Bergy's Manual of Systematic Bacteriology. Vol. I, 275-278 [1984]; F. Gosselé et al., International J. System. Bacteriol. 33, 65-81 [1983]). Suitable Acetobacter hosts for the shuttle vectors of the present invention are any strains belonging to genus Acetobacter. Preferred strains of the genus Acetobacter are Acetobacter aceti, Acetobacter liquefaciens and Acetobacter pasteurianus.

The vectors of the present invention containing an insert DNA can be maintained stably in the above microorganisms, specially in Gluconobacter strains, even without a selective pressure with antibiotics and the like for a simple and economical industrial process.

It is also very useful that the vectors of the present invention having an origin functional in Escherichia coli and an origin functional in Gluconobacter oxydans are functional both in Escherichia coli and Gluconobacter and Acetobacter strains. Escherichia coli is known to be an efficient host for amplification of a vector DNA and manipulation of recombinant DNA by simple and rapid methods. On the other hand, Gluconobacter can be used as a host for expression of Gluconobacter genes. Since the vectors of the present invention are such functional constructs, they enable cloning of certain genes of Gluconobacter or Acetobacter in Escherichia coli and thereafter the effective expression in Gluconobacter or Acetobacter. Furthermore, it is favorable that such functional constructs also contain a DNA region necessary for conjugal transfer (Mob site). Hence the vectors of the present invention can be firstly assembled in Escherichia coli

3

and then directly introduced into Gluconobacter or Acetobacter by cunjugal mating without isolation of plasmid DNA from Escherichia coli.

Suitable marker genes for the shuttle vectors of the present invention, are all antibiotic resistance genes which are expressed in Escherichia coli, Gluconobacter or Acetobacter, such as kanamycin resistance (Km$^r$), streptomycin resistance (Sm$^r$), ampicillin resistance (Ap$^r$) and tetracycline resistance (Tc$^r$).

These antibiotic resistance genes may be isolated from natural or artificially-constructed plasmids, transposons, chromosomal DNAs and synthetic DNAs. Specific sources for the antibiotic resistance genes include but are not limited to plasmids RP4 (Datta et al., J. Bacterial. 108, 1244-1249 [1971]; NRRL B-18147), RK2 (ATCC 37125), RSF1010 (Nagahari and Sakaguchi, J. Bacteriol. 133, 1527-1529 [1978]; NRRL B-18146), pACYC177 (ATCC 37031), transposons Tn3 (Berg et al., Proc. Nat. Acad. Sci. 72, 3628-3632 [1975]), Tn10 (Foster et al., J. Bacteriol. 124, 1153-1158 [1975]) and their derivatives. As marker genes, pigment-producing genes (e.g. the mel gene on pIJ702) may also serve.

Suitable replication origins functional in Escherichia coli for the shuttle vectors of the present invention are, e.g., DNA fragments containing a replication origin of Escherichia coli, or of any plasmid or phage which can autonomously replicate in Escherichia coli. Such replication origins may be isolated, e.g. from plasmids RP4, RSF1010, pBR322, pACYC177, pACYC184, pSC101, λ phages (e.g. phage λ, P1 phages (e.g. P1) or T-coliphages (e.g. coliphage T4).

Suitable replication origins functional in Gluconobacter and Acetobacter for the shuttle vectors of the present invention, are e.g., DNA fragments containing a replication origin of Gluconobacter oxydans, or of any endogenous plasmid or phage which can autonomously replicate in Gluconobacter oxydans. Such replication origins may be isolated, e.g. from Gluconobacter oxydans IFO 3293 (FERM P-8356), the endogenous plasmids of Gluconobacter oxydans IFO 3293, or from endogenous Gluconobacter oxydans phage DNAs (Schocher et al., Arch. Microbiol. 121, 193-197 [1979]).

The Mob site is believed to include the origin of transfer (ori T) and to act as a recognition site for certain transactive plasmid's transfer functions (R. Simon et al., Bio/Technology 1, 784-791 [1983]). The Mob site can be obtained from a conjugative plasmid, e.g., plasmids RK2, RP4, RSF1010 or their derivatives. The Mob site-containing plasmid can be transferred from its original host to another host with the help of Tra genes function by using bi-parental conjugal mating or tri-parental conjugal mating. The Tra genes are well known as transfer genes of the broad-host-range Incp-tpye plasmids, such as plasmids RP4 and RK2. In the bi-parental conjugal mating, Tra genes-containing strains, e.g., E. coli S17-1 (R. Simon et al., supra) harboring Mob site-containing plasmids are mixed with a recipient strain. In the tri-parental conjugal mating, a donor strain harboring Mob site-containing plasmids is mixed with a strain harboring plasmids containing Tra genes, such as RP4 and RK2, and with a recipient strain.

The shuttle vectors of the present invention may also comprise one or more inserts, for example, DNA sequences having multicloning sites, expression control sequences, cos sites, terminator sequences, ribosome binding sites, DNA sequences encoding signal peptides and/or proteins, to add further desirable functions to the shuttle vector.

In more detail, the shuttle vectors of the present invention may comprise DNA sequences including one or more multicloning sites (Messing et al., Methods in Enzymology, 101, 20 [1983]) derived from a variety of plasmids and phages, such as pUC18 (Boehringer Mannheim) or M13mp8 (Boehringer Mannheim), or from synthetic DNA sequences for convenient cloning. Furthermore, the shuttle vectors may contain a wide variety of expression control sequences, such as the lac, trp, tac, or β-lactamase expression control system, control sequences of phage origin, which are, e.g., known from the textbook of Maniatis et al. (Molecular cloning, A Laboratory Manual, Cold Spring Harbor Press, Cold Spring Harbor, N.Y., USA [1982]) or expression control sequences derived from Gluconobacter strains. In addition, the shuttle vectors may contain cos sites for in vitro packaging. Furthermore, they may contain terminator sequences for effective termination, natural or synthetic ribosome binding sites for effective translation, DNA sequences encoding signal peptides for efficient localization of the cloned protein(s) and structural genes of marker proteins all of which are used to construct the efficient Gluconobacter and Acetobacter host-vector systems of the present invention.

Briefly, the shuttle vectors of the present invention can be obtained by the following steps using the materials as described in the present description and by using recombinant DNA techniques as described by Maniatis et al. (supra):

(1) Preparing a DNA containing marker genes.

(2) Preparing a DNA containing a replication origin functional in Escherichia coli.

(3) Preparing a DNA containing a replication origin functional in Gluconobacter.

(4) Preparing a DNA containing a Mob site.

(5) Combining the DNAs described in (1) to (4) by digesting the said DNAs with an appropriate restriction enzyme and ligating them to obtain the recombinant shuttle vectors of the present invention.

By these steps, the shuttle vectors containing marker genes, two different replication origins, one functional in Escherichia coli and one from Gluconobacter and a functional Mob site can be constructed.

The shuttle vectors of the present invention can be transferred from Escherichia coli to Gluconobacter or Acetobacter with a very high frequency (about $10^{-2}$ to $10^{-1}$ transconjugants per recipient) by a conjugal mating without the isolation and purification of the vector DNA. With these vectors, a genomic library constructed in Escherichia coli ($10^3$ - $10^5$ clones) can be transferred into Gluconobacter or Acetobacter in one experiment. Thus, the shuttle vectors of the present invention are highly efficient in viewpoint of simplicity in a cloning experiment.

The most advantageous characteristic of the shuttle vectors of the present invention is their stability in Escherichia coli, Gluconobacter and Acetobacter strains even in the absence of selective pressure. Furthermore, the said vectors affect neither the growth of host cells nor the formation of fermentation products.

In summary, the shuttle vectors of the present invention can be used in industrial processes as vectors for gene cloning and for the production of desired pro- and eukaryotic polypeptides. The desired pro- and eukaryotic polypeptides can be obtained by introducing by transconjugation into a strain of genus Gluconobacter or genus Acetobacter a shuttle vector of the present invention containing the DNA sequence coding for said polypeptide operatively linked with an expression control sequence, cultering the transconjugant under appropriate conditions of growth and isolating the desired polypeptide from the culture.

## Brief description of the drawings

Figure 1 illustrates the construction of the shuttle vector, pGE1.

Figure 2 illustrates the insertion of the membrane-bound L-sorbosone dehydrogenase gene into plasmid pGE1.

Figure 3 represents the restriction maps of plasmids pGE1 and pGE-SNB2.

Figure 4 illustrates the restriction maps of plasmids p7A6Δ2, p7A6Δ3 and p7A6Δ4.

Figure 5 illustrates the restriction map of the SSE fragment of plasmid p7A6Δ4.

## Example 1

### Construction of the shuttle vector pGE1

Gluconobacter oxydans (G. oxydans) IFO 3293 contains two kinds of criptic plasmids which are maintained stably in the cell without any selective pressure. The smaller plasmid designated pGO3293S is a relaxed type plasmid (the copy number is more than 10) and its molecular size is 9.9kb. The larger plasmid designated pGO3293L is a stringent type plasmid (the copy number is 1 to 2) and its molecular size is about 60kb. Considering the appropriate copy number and DNA size of the said vectors pGO3293S was selected as source for the replication origin functional in G. oxydans.

Plasmid pSUP301 was used as source for the replication origin functional in Escherichia coli (E. coli), the antibiotic resistance genes (Km$^r$ gene and Ap$^r$ gene) and the Mob site. Plasmid pSUP301 was constructed by combining pACYC177 with a Mob site derived from RK2 (R. Simon et al., supra). Therefore, plasmids pACYC177 and RK2, or the like, can be used in place of plasmid pSUP301.

### A) Preparation of plasmids pGO3293S and pSUP301

Plasmid pSUP301 was prepared from E. coli cells harboring plasmid pSUP301 by the alkaline method (H.C. Birnboim and J. Doly, Nucleic Acids Research, 7, 1513-1523 [1979]).

Plasmid pGO3293S was prepared from G. oxydans IFO 3293 cells by the alkaline method as described below:

G. oxydans cells were cultivated in a test tube containing 5 ml of mannitol broth (MB) medium containing 25 g/l mannitol, 5 g/l yeast extract (Difco), and 3 g/l Bactopeptone (Difco) for 24 hours at 30°C. Then, 4 ml of the culture broth were transferred into a 500 ml Erlenmeyer flask containing 50 ml of fresh MB medium. The flask was incubated at 30°C for 15 hours on a rotary shaker operating at 180 rpm. The resulting broth was centrifuged at 5,000 rpm (3,000 g) for 15 minutes. The cells were suspended in 10 ml of solution I (25 mM Tris-HCl, pH 7.9, containing 2 mg/ml lysozyme, 5 mM glucose, 10 mM EDTA). The cell suspension was kept on ice for 30 minutes. Then, 20 ml of solution II (18 mM NaOH containing 1% SDS)

5

were added. The solution was kept on ice for 10 minutes and added with 15 ml 3M sodium acetate, pH 4.8. The resulting solution was kept on ice for 60 minutes and was centrifuged at 13,000 rpm (21,000 g) for 10 minutes. 40 ml of isopropanol were added to the supernatant (40 ml). The mixture was kept on ice for 60 minutes and centrifuged at 15,000 rpm (28,000 g) for 15 minutes. The pellet was dried and dissolved in 2 ml of distilled water. The plasmid DNA was further purified by ethanol precipitation to obtain a final plasmid DNA solution.

B) Construction of a composite plasmid using plasmids pGO3293S and pSUP301

150 ng of pSUP301 DNA and 200 ng of pGO3293S DNA were completely digested with HincII and ligated with T4 DNA ligase. The resulting ligation mixture was used to transform E. coli S17-1.

Km$^r$ transformants were first selected on LK (Luria broth containing 50 $\mu$g/ml kanamycin; Luria broth (LB): 10 g/l bactotryptone, 5 g/l yeast extract, 5 g/l NaCl) agar plates. Then, Ap$^s$Km$^r$ clones, i.e. transformants carrying a plasmid with an insert in the Ap$^r$ gene, were selected using both LK agar plates and LA (LB medium containing 50 $\mu$g/ml ampicillin) agar plates.

C) Transfer of composite plasmids from E. coli to G. oxydans by bi-parental conjugation

A mixture of composite plasmids obtained from all the Km$^r$, Ap$^s$ transformants were transferred to G. oxydans N44-1 (a 2-KGA high producer mutant strain obtained from G. oxydans IFO 3293 by mutagenesis as described in European Patent Application, Publication No. 213 591) by a bi-parental conjugal mating. Bi-parental conjugal mating was carried out as follows:

Two hundred $\mu$l of a log phase culture of the recipient G. oxydans N44-1 grown in MB medium were mixed with 100 $\mu$l of log phase culture of all the Km$^r$ transformants grown in LB medium containing 5 $\mu$g/ml kanamycin and 10 $\mu$g/ml streptomycin and spotted onto nitrocellulose filter on the surface of FB (50 g/l fructose, 10 g/l yeast extract, 10 g/l polypeptone) agar plates. The plates were incubated overnight at 30°C. The mixed colonies were plated onto MB containing 10 $\mu$g/ml polymyxin B and 50 $\mu$g/ml kanamycin (MPK) agar plates, and MB containing 10 $\mu$g/ml polymyxin B (MP) agar plates after an appropriate dilution, and incubated for 4 days at 30°C.

The frequency of the conjugation was calculated as a ratio of number of colonies on MPK agar plates to number of colonies on MP agar plates.

The frequency of this experiment was 0.06-0.1% (transconjugant per recipient). The plasmid DNAs were prepared from 5 transconjugants by mini-alkaline method and analyzed by agarose gel electrophoresis. Consequently, all the plasmids tested had the same structure. One plasmid was designated pGE1.

D) Characterization of the newly-isolated plasmid pGE1

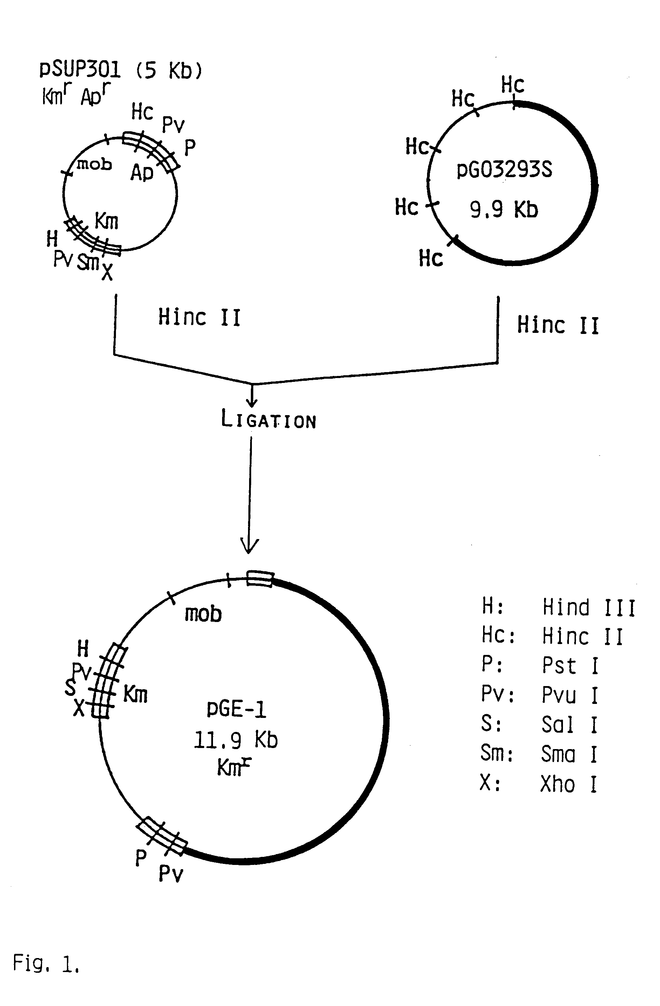

Plasmid pGE1, prepared from cells of G. oxydans N44-1 harboring pGE1, was introduced into E. coli strain C600 by transformation to confirm if it is a shuttle vector between G. oxydans and E. coli or not. E. coli strain C600 was transformed with plasmid pGE1 at a frequency of 10$^6$ transformants per $\mu$g DNA. This finding indicated that pGE1 worked as a shuttle vector between E. coli and G. oxydans. The schematic presentation of construction of pGE1 is shown in Fig. 1.

pGE1 DNAs prepared from cells of G. oxydans N44-1 harboring pGE1 and E. coli S17-1 were digested with AvaI, HincII, PstI, PvuI or PvuII and were analyzed by agarose gel electrophoresis. The DNA fragments were Southern-transferred to nitrocellulose filters. The HincII fragments, PstI fragments and non digested DNA were hybridized with $^{32}$P-labeled pSUP301, while the PvuI, PvuII and AvaI fragments were hybridized with $^{32}$P-labeled pGO3293S.

The autoradiogram clearly showed that pGE1 contains the DNA fragments derived from both pSUP301 nd pGO3293S and that pGE1 exists in the same structure in both E. coli and G. oxydans. Neither modifications no deletions were noted.

Example 2

Conjugal transfer of plasmid pGE1 and other plasmids into G. oxydans

Efficiencies of conjugal transfer of various vectors were compared to characterize plasmid pGE1.

Five plasmids, viz. RP4, RSF1010, pVK102 (ATCC 37158), pSUP301 and pGE1, were transferred from E. coli to G. oxydans N44-1 using the conjugal transfer system. The best frequency of the conjugal transfer of each plasmid is shown in Table 1. Plasmid pSUP301 could not be introduced into Gluconobacter because a replication origin for Gluconobacter is absent in this plasmid. On the other hand, plasmid pGE1 as well as plasmids RP4, RSF1010, pVK102 were transferred into Gluconobacter at the high frequency of $10^{-2}$ to $10^{-1}$ (transconjugants per recipient).

Table 1

| Frequency of the conjugal transfer | | |
|---|---|---|
| Vector | Mwt (kb) | Frequency (%) (Transconjugants/recipient) |
| RP4 | 56 | $10^{-2}$ - $10^{-1}$ |
| RSF1010 | 8.9 | $10^{-1}$ |
| pVK102 | 23 | $10^{-2}$ - $10^{-1}$ |
| pSUP301 | 5 | $<10^{-6}$ |
| pGE1 | 11.0 | $10^{-1}$ |

Example 3

Effect of plasmid pGE1 on cell growth and 2-KGA production

New plasmid pGE1 and plasmids RP4, RSF1010 and pVK102 were introduced into G. oxydans N44-1 as described in the Example 1-(C). The transconjugants were transferred from MB agar plates containing antibiotics into test tubes containing 5 ml of No. 5 medium (80 g/l L-sorbose, 0.5 g/l glycerol, 15 g/l yeast extract, 2.5 g/l $MgSO_4 \cdot 7H_2O$, 15 g/l $CaCO_3$) with or without antibiotics. The test tubes were incubated on a shaker at 30°C for 5 days. N44-1 was also cultivated in the No. 5 medium in the same way as a control but without antibiotics. The fermentation broth was assayed for cell growth and 2-KGA production by determining the optical density at 550 nm ($OD_{550}$), and the 2-KGA amount.

As shown in Table 2, plasmid pGE1 is the best vector for the maintenance of growth and 2-KGA production among the vectors tested. In contrast, the presence of RP4 caused the reduction of both cell growth (by 20%) and 2-KGA production (by 45%). In addition, another defect was found in RP4; deletion of RP4 occurred when it replicated in Gluconobacter.

RSF1010 and pVK102 did not affect the growth but often lowered the 2-KGA productivity of N44-1 to 80% and 90% of the level of the standard, respectively.

7

## Table 2

### Effect of the presence of various vectors on growth and 2-KGA production

| Host | Vector | OD550 Antibiotic * | | 2-KGA (g/l) | |
|------|--------|-----|-----|-----|-----|
| | | + | − | + | − |
| | − | − | 21.1 | − | 52.1 |
| | RP4 | 14.1 | 15.9 | 26.0 | 31.8 |
| N44-1 | RSF1010 | 20.4 | 20.8 | 42.7 | 43.3 |
| | pVK102 | 20.1 | 20.5 | 48.8 | 48.1 |
| | pGE1 | 20.2 | 20.8 | 54.4 | 55.9 |

\* Antibiotic for RP4, pVK102 and pGE1 was kanamycin
(50 µg/ml) and that for RSF1010 was streptomycin
(50 µg/ml).

Example 4

Stability of plasmid pGE1 during cultivation

N44-1-transconjugants described in Example 3 were cultivated in Km-free broth, diluted and plated on MB agar plates. The plates were incubated a 30°C for 5 days. E. coli strain S17-1 harboring plasmid pGE1 was cultivated in Km-free LB medium, diluted and plated on LB agar plates. The plates were incubated a 37°C for 1 day.

The resulting colonies were used to determine the stability of the plasmids as follows:

Plasmid stability was defined as the ratio of the number of colonies grown on agar plates containing antibiotics to that of colonies grown on agar plates without antibiotics.

Liquid samples withdrawn from the culture broth at various stages of cultivation were appropriately diluted, plated on antibiotics-free agar plates and incubated at 37°C for one day (E. coli) or at 28°C for five days (G. oxydans). One hundred colonies grown on these plates were transferred onto agar plates with and without antibiotics. The plates were incubated in the same conditions as described above and the number of colonies which appeared was counted for the calculation of plasmid stability.

Table 3-(a) shows that RP4 was extremely unstable in the 2-KGA high producer N44-1 but stable in the 2-KGA non-producer C20 (a mutant strain obtained from G. oxydans IFO 3293 by mutagenesis as described in European Patent Application, Publication No. 213 591). The other vectors were considerably stable in both strains.

However, in our previous experiments, it was noted that RSF1010 with an insert was often cured or deleted (data not shown). Therefore, pGE1 and pVK102 were further studied concerning the stabilities during cultivation.

N44-1 cells harboring plasmids pVK102, p7A6Δ4 (derivative of pVK102 containing the gene for membrane-bound sorbosone dehydrogenase; for construction see Example 8) or pGE1 were cultivated in MB liquid medium with 50 µg/ml kanamycin in test tubes for 2 days to prepare a seed culture. One tenth ml of the seed culture was transferred to 5 ml of Km-free MB medium in a test tube. The test tube was incubated at 30°C for 1 day on a tube shaker. Three cycles of this transfer and cultivation were carried out in MB medium. One tenth ml of the same seed culture was also transferred to 5 ml of Km-free No. 5 medium in a test tube to test plasmid stability under 2-KGA producing condition. Three cycles of transfer and cultivation (at 30°C for 2 to 3 days) were also carried out in No. 5 medium. At the end of each cultivation, the broth was appropriately diluted and plated on MB agar plates to determine the stability of the plasmid used.

Table 3-(b) shows that plasmid pGE1 was extremely stable (100%) under the examined conditions, while pVK102 and p7A6Δ4 were considerably, but to a lesser extent, stable (74-100%). These results indicate that the 2-KGA fermentation can be carried out by the 2-KGA high producer harboring pGE1 without the addition of kanamycin in the practical process which includes 2 to 3 cycles of seed cultivations. Plasmid pGE1 was also very stable (100%) in E. coli during three cycles of cultivations without antibiotics.

Table 3-(a)

| Stability of various vectors during cultivation Stability during cultivation in No. 5 medium (%) | | | | |
|---|---|---|---|---|
| Host | Vector | | | |
| | RP4 | RSF1010 | pVK102 | pGE1 |
| | a) (Km$^r$) b) (Tc$^r$) | (Sm$^r$) | (Km$^r$) | (Km$^r$) |
| N44-1 | a) 18% b) 18% | 100% | 78% | 90% |
| C-20 | a) 100% b) 100% | 89% | 84% | NT |

Table 3-(b)

| Stability during cultivation in MB medium (%) and No. 5 medium (%) | | | |
|---|---|---|---|
| Plasmid | Transfer No. | Plasmid stability in: | |
| | | MB medium | No. 5 medium |
| pVK102 | 1 | 98 | 96 |
| | 2 | 96 | 94 |
| | 3 | 90 | 74 |
| p7A6Δ4 | 1 | 98 | 92 |
| | 2 | 100 | 100 |
| | 3 | 94 | 88 |
| pGE1 | 1 | 100 | 100 |
| | 2 | 100 | 100 |
| | 3 | 100 | 100 |

N44-1-harboring each plasmid was cultivated in mannitol broth (MB) for 1 day or in No. 5 medium for 2 to 3 days and 2% of the broth was transferred into each fresh medium.

Example 5

Compatibility of plasmid pGE1 with plasmid pVK102

Compatibility of plasmid pGE1 with plasmid pVK102 was examined since the introduction of 2-KGA fermentation-relating genes into one host strain will presumably require two or more vectors which are compatible.

Plasmid pVK102 was transferred by bi-parental conjugal mating from E. coli S17-1 to G. oxydans IFO 3293 which harbored plasmid pGE1. Transconjugants were selected on the basis that the bacteria showed both kanamycin- and tetracycline-resistances. Plasmid DNAs prepared from Km$^r$ and Tc$^r$ transconjugants were used to transform E. coli S17-1 to confirm the presence of both plasmids in the transconjugants. Among the transformants, there were pGE1 and pVK102-harboring ones as expected. These findings indicate that plasmid pGE1 is compatible with plasmid pVK102 in Gluconobacter.

Example 6

Host range of plasmid pGE1

Plasmid pGE1 was conjugal-transferred from E. coli S17-1 into various strains belonging to genus Gluconobacter, viz. G. oxydans IFO3293, G. oxydans IFO3462, G. oxydans IFO3268, G. oxydans IFO3271, G. oxydans IFO3287, G. oxydans IFO 3172 and G. oxydans ATCC9937 and into various strains belonging to genus Acetobacter, viz. A. acetosus IFO3129, A. pasteurianus IFO3170, A. pasteurianus IFO3223 and A. pasteurianus IFO3225 by a bi-parental conjugal mating as described in Example 1(C).

Km$^r$ colonies were obtained from all strains of Gluconobacter and Acetobacter. Then, the plasmid DNAs of obtained Km$^r$ strains were analyzed for their characteristics in comparison with the original plasmid pGE1.

The plasmid DNAs were subjected to agarose gel electrophoresis. The analysis showed that all the plasmids had the same molecular size as plasmid pGE1. The DNA bands on agarose gel were transferred to nylon filter and subjected to Southern-hybridization analysis. All the DNA bands hybridized with $^{32}$P-labeled plasmid pGE1 DNA. Thus, all the Km$^r$ strains obtained from the strains of Gluconobacter and Acetobacter were confirmed to be transconjugants harboring plasmid pGE1.

Plasmid DNAs isolated from each transconjugant were used to tranform E. coli strain C600. Km$^r$ transformants obtained were cultivated in LK liquid medium at 37°C one overnight. Plasmid DNA was extracted from each tranformant and analyzed by agarose gel electrophoresis after digestion with Bgl II. All plasmid DNAs examined were confirmed to be identical with plasmid pGE1 DNA. Neither deletion nor insertion of DNA were observed. Consequently, plasmid pGE1 works as a shuttle vector for various Gluconobacter and Acetobacter strains.

Example 7

Stability of plasmid pGE1 in various strains belonging to genus Gluconobacter and Acetobacter

Every transconjugant described in Example 6 maintained on MB agar plate containing kanamycin (50 $\mu$g/ml) was transferred into MB liquid medium containing 50 $\mu$g/ml kanamycin in a test tube and cultivated for 2 days to prepare a seed culture. One tenth ml of the seed culture was transferred to 5 ml of kanamycin-free MB medium. The test tube was incubated at 30°C for 1 day on a tube shaker. Three cycles of this transfer and cultivation were carried out in MB medium. The seed culture, first culture and third culture were appropriately diluted and spread on MB agar plates to determine the stability of the plasmid pGE1 as shown in Example 4.

Table 4 shows that plasmid pGE1 was extremely stable (100%) in G. oxydans IFO3293, G. oxydans IFO3268 and A. acetosus IFO3129 and considerably stable (68-98%) in G. oxydans IFO3462, G. oxydans IFO3271, G. oxydans IFO3287, G. oxydans IFO3172, G. oxydans ATCC9937, A. pasteurianus IFO3170, A. pasteurianus IFO3223 and A. pasteurianus IFO3225 even after three transfers without kanamycin selection.

Table 4

| Stability of plasmid pGE1 in various strains belonging to genus Gluconobacter and genus Acetobacter | | | | |
|---|---|---|---|---|
| | | Plasmid-harboring strains (%) | | |
| | | in seed cul.[a] | 1st cul.[b] | 3rd cul.[b] |
| G. oxydans | IFO 3293 | 100 | 100 | 100 |
| " | IFO 3462 | 100 | 94 | 88 |
| " | IFO 3268 | 100 | 100 | 100 |
| " | IFO 3271 | 86 | 82 | 68 |
| " | IFO 3287 | 100 | 100 | 94 |
| " | IFO 3172 | 96 | 98 | 96 |
| " | ATCC 9937 | 100 | 96 | 92 |
| A. acetosus | IFO 3129 | 100 | 100 | 100 |
| A. pasteurianus | IFO 3170 | 96 | 76 | 86 |
| " | IFO 3223 | 100 | 98 | 96 |
| " | IFO 3225 | 100 | 98 | 100 |

a) Seed culture was carried out in MB medium containing 50 $\mu$g/ml kanamycin.

b) First and third cultures were carried out in MB medium without kanamycin.

Example 8

Use of plasmid pGE1 as a cloning vector

A) Integration of membrane-bound L-sorbosone dehydrogenase gene into plasmid pGE1

The cloned gene of membrane-bound L-sorbosone dehydrogenase of A. liquefaciens IFO 12258 was introduced into plasmid pGE1 to demonstrate its usefulness as a cloning vector.

The BglII fragment (5.5kb) containing membrane-bound L-sorbosone dehydrogenase (SNDH) gene was isolated from plasmid p7A6Δ4 and ligated with plasmid pGE1 partially digested by BglII. The resulting DNA mixture was used to transform E. coli S17-1. Five hundred (500) transformants were picked and transferred onto nitrocellulose filters placed on the surface of LSK (LB medium containing 100 $\mu$g/ml streptomycin and 50 $\mu$g/ml kanamycin) agar plates and incubated at 37°C overnight. The filters were treated with 0.5M NaOH - 1.5M NaCl solution for 5 minutes and neutralized with 3M sodium acetate (pH 4.8) for 5 minutes. Colony-blotted filters were used for hybridization with $^{32}$P-labeled 1.8kb SalI fragment which was isolated from p7A6Δ4 and contained only DNA of A. liquefaciens IFO 12258. Fifteen positive clones were selected and their plasmids were analyzed by digestion with BglII or PvuI.

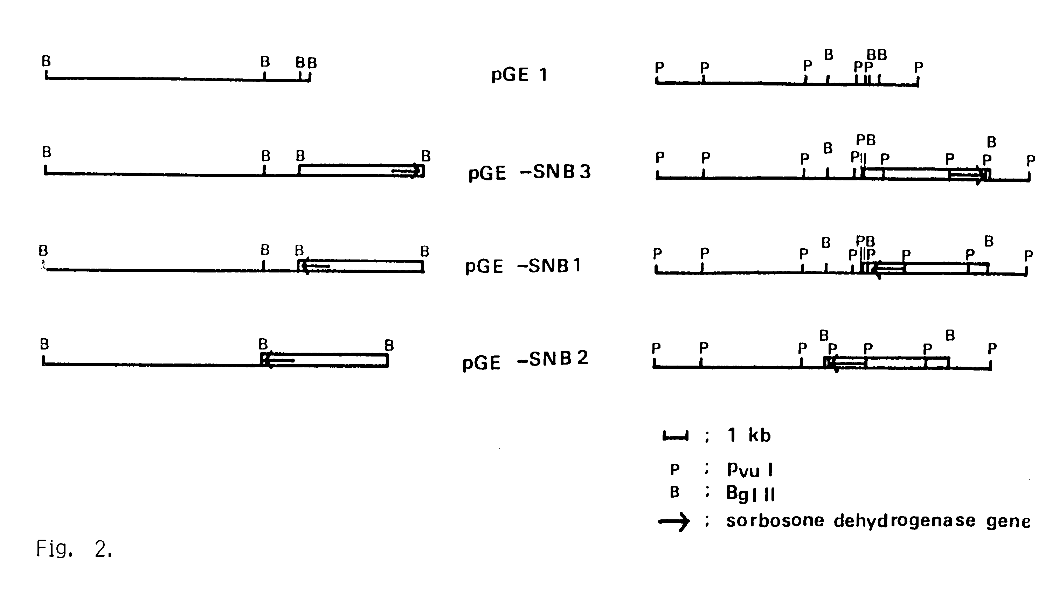

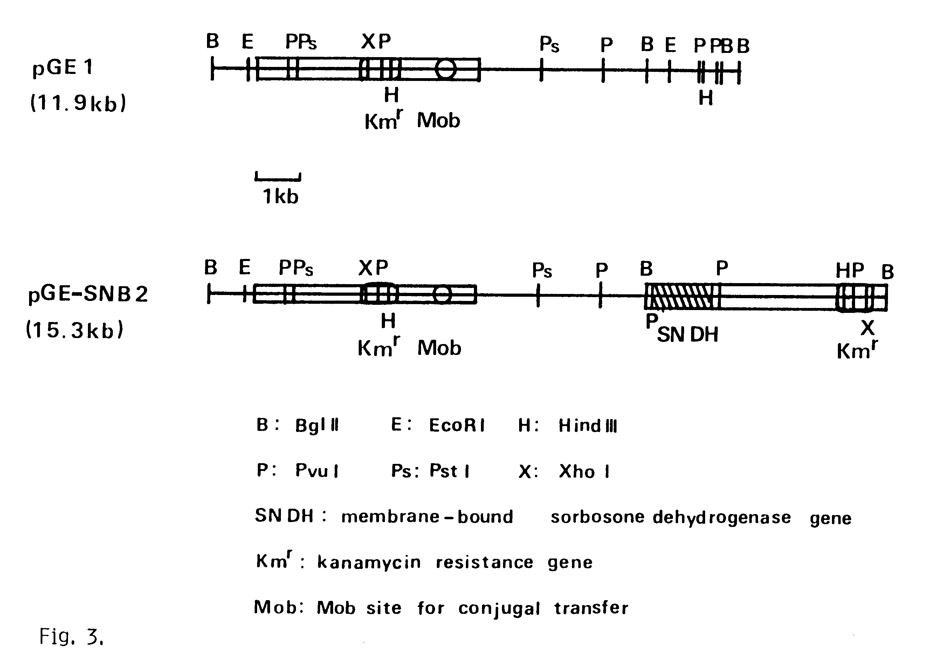

As shown in Fig. 2, three types of subclones were isolated which are hereafter referred to as pGE-SNB1, pGE-SNB2 and pGE-SNB3. All of them contained a 5.5kb BglII fragment. Simultaneously, they, however, lost 1.7kb and/or 0.4kb BglII fragments from plasmid pGE1, which consists of 3 BglII fragments (9.8kb, 1.7kb and 0.4kb). Therefore, plasmid pGE1 can be shortened to 9.8kb as a viable vector by BglII digestion. The detailed restriction maps of pGE1 and pGE-SNB2 are shown in Fig. 3.

B) Effect of subclones on 2-KGA production in a resting system

The effect of SNDH gene-containing pGE1 derivatives carried in N44-1 on 2-KGA production was examined in comparison with that of plasmids pGE1, pVK102 or p7A6Δ4 carried in N44-1. Two natural strains possessing an efficient membrane-bound L-sorbosone dehydrogenase, A. liquefaciens (ATCC 23750) and Pseudomonas putida (P. putida) (ATCC 21812; Makover et al., Biotechnol. Bioeng. 17, 1485-1514 [1975]) were also used as control strains.

Three subclones of SNDH gene-containing plasmids pGE-SNB1, pGE-SNB2 and pGE-SNB3, as well as plasmids pGE1, pVK102 and p7A6Δ4 were transferred from E. coli S17-1 into G. oxydans IFO 3293 by bi-parental conjugal mating. The transconjugants, A. liquefaciens (ATCC 23750) and P. putida (ATCC 21812) were cultivated in MB overnight. Cells were then diluted to an $OD_{550}$ = 2 and cells collected from 4 ml of the cell suspension were resuspended in 4 ml of reaction mixture containing 18 g/l L-sorbosone, 10 g/l

CaCO$_3$ and 3 g/l NaCl and incubated at 30°C. Table 5 shows the amounts of 2-KGA produced after 2, 20 and 63 hours.

The 2-KGA productivities of transconjugants containing SNDH gene containing subclones pGE-SNB1, pGE-SNB2 and pGE-SNB3 were better than those of A. liquefaciens (ATCC 23750) and P. putida (ATCC 21812). Furthmore, 2-KGA production by these subclones was almost as good as 2-KGA production of plasmid p7A6Δ4.

When the said subclones were introduced into N44-1, a 2-KGA high producer, they showed better 2-KGA yields than N44-1 either from L-sorbose or L-sorbosone under the resting system as shown in Table 6. Molar yield of 2-KGA from L-sorbosone by strain with SNDH gene-containing plasmid was 90%, whereas molar yield by strain with a vector plasmid only was 20%. Molar yield of 2-KGA from L-sorbose by SNDH gene-containing strain was 60 to 70% whereas that of SNDH gene-free strain was about 40%.

Table 5

| Comparison of 2-KGA production rate from L-sorbosone under a resting system | | | | |
|---|---|---|---|---|
| | | 2-KGA (g/l) / 2 OD$_{550}$ | | |
| | | 2 hr. | 20 hr. | 63 hr. |
| G. oxydans IFO 3293 | - | 0.0 | 2.1 | 1.9 |
| | pVK102 | 0.0 | 1.3 | 1.1 |
| | p7A6Δ4 | 1.6 | 15.8 | 19.1 |
| | pGE1 | 0.0 | 1.5 | 3.2 |
| | pGE-SN B1 | 1.8 | 18.4 | 19.4 |
| | pGE-SN B2 | 1.3 | 10.7 | 15.4 |
| | pGE-SN B3 | 1.8 | 17.2 | 19.2 |
| A. liquefaciens ATCC 23750 | - | 0.9 | 6.4 | 8.9 |
| P. putida ATCC 21812 | - | 0.9 | 7.4 | 10.0 |
| L-Sorbosone: 18 g/l Temperature: 30°C | | | | |

Table 6

| 2-KGA production from L-Sorbosone or L-Sorbose by N44-1 carrying various plasmids under a resting system | | | |
|---|---|---|---|
| | | 2-KGA produced (g/l) from | |
| | | 36 g L-Sorbosone | 40 g/l L-Sorbose |
| G. oxydans N44-1 | - | 13.0 | ND |
| | pVK102 | 7.6 | 19.9 |
| | p7A6Δ4 | 33.8 | 31.2 |
| | pGE1 | 8.5 | ND |
| | pGE-SN B1 | 31.8 | 30.3 |
| | pGE-SN B2 | 35.5 | 25.4 |
| ND: Not determined | | | |

Reaction from L-sorbosone or L-sorbose was carried out at 30°C for 1 day or for 6 days, respectively.

Example 9

Construction of plasmid p7A6Δ4

(A) Construction of a cosmid genomic library in E. coli S17-1

G. oxydans IFO 12258 was cultivated in 200ml of mannitol broth (MB) (25 g/l of mannitol, 3 g/l of bactopeptone, 5g/l of yeast extract) for 48 hours at 30°C. The cells were collected by centrifugation, washed with 100ml of Tris (10mM)-EDTA (1mM) buffer, pH 7.5 and resuspended in 50ml of Tris (10mM)-EDTA (20mM) buffer, pH 7.5.

The cell suspension thus prepared was treated with 2ml of the lysozyme solution (10mg/ml) at 37°C for 30 minutes followed by the treatment with pronase (4000 units) at 37°C for 30 minutes and 10ml of 5% SDS at 37°C for 1 hour. At this point, a clear lysate was obtained. DNA was extracted with 60ml of neutral phenol : chloroform containing 4% octanol (1:1) by rotating slowly at 4°C for 30 minutes. The mixture was centrifuged at 15000rpm (28.000 g) for 15 minutes and the supernatant obtained was extracted with 60ml of chloroform : octanol (96:4) by rotating slowly at 4°C for 10 minutes.

To the 50ml supernatant obtained by centrifugation at 15000rpm (28.000 g) for 15 minutes were added 5ml of 3M sodium acetate and 55ml of cold ethanol. The crude DNA was obtained by winding out with a glass rod, which was then treated with RNase T1 and A (37°C, 30 minutes) and pronase (37°C, 30 minutes) again. The phenol and chloroform extractions were repeated to obtain pure chromosomal DNA.

The chromosomal DNA of G. oxydans IFO 12258 was partially digested with Sal I. The resulting fragments of 15kb to 35kb were isolated from agarose gel by electrophoresis. Plasmid pVK102 (ATCC 37158) DNA was completely digested with Sal I and dephosphorylated with calf intestine alkaline phosphatase. The DNA fragments of 15kb to 35kb and the linear pVK102 DNA were ligated with T4 DNA ligase.

The ligated fragments were used for in vitro packaging using a packaging kit (Amersham International plc) and the resulting phage particles were incubated with E. coli ED8767 (Murray et al., Mol. Gen. Genet. 150, 53, 1977). The cell suspension was plated on to LB agar plates containing 50μg/ml kanamycin.

One thousand Km$^r$ colonies were scraped and used to prepare a mixture of recombinant plasmids. The plasmid DNAs were used to transform E. coli S17-1 (Sm$^r$ Tra$^+$). One thousand and four hundred transformants were picked into microtiter plates containing LB medium with 100mg/ml of streptomycin and 50μg/ml of kanamycin, incubated overnight and stored with 15% glycerol at -80°C as the genomic library of G. oxydans IFO 12258 in E. coli S17-1. The average size of the inserts was 25kb to 30kb.

(B) Conjugal transfer of the cosmid genomic library in E. coli S17-1 into G. oxydans OX-4

The cosmid genomic library of G. oxydans IFO 12258 in E. coli S17-1 was transferred to G. oxydans OX-4 (a L-sorbosone accumulating mutant obtained from G. oxydans IFO 3293 by mutagenesis as described in European Patent Application, Publication No. 213 591) using bi-parental mating between both strains.

Two hundred μl of log phase culture of the recipient G. oxydans OX-4 grown in mannitol broth were mixed with 100μl of log phase culture of every E. coli S17-1 carrying pVK102 with insert DNA individually and spotted onto nitrocellulose filter on the surface of fructose broth (50g/l fructose, 5g/l yeast extract, 5g/l polypeptone) agar plates. The plates were incubated overnight at 30°C. The mixed colonies were streaked onto MB containing 10μg/ml polymyxin B and 50μg/ml kanamycin (MPK) agar plates and incubated for 4 days at 30°C. The resulting transconjugants were purified by restreaking on MPK agar plates.

Screening was carried out using a mini-resting system. About 1400 strains of G. oxydans OX-4/pVK102 with insert DNA were suspended in the reaction mixture containing 50μl of 3g/l of NaCl, 10g/l of CaC0$_3$ and 30g/l of L-sorbose or L-sorbosone individually and incubated for 1 to 5 days at 30°C. Assays for 2-KGA production were performed by thinlayer chromatography on silica gel. One positive clone, p7A6, was obtained.

The recombinant plasmid p7A6 was prepared by the alkaline method of Birnboim and Doly (Nucleic Acids Research 7, 1513-1523 [1979]) and fragmented with the following restriction enzymes:

EcoRI, EcoRV, HaeIII, HincII, NruI, Sal I, Sau 3A, XhoII, BamHI, BglII, Dra I, Hind III and Sma I. EcoRI, HincII, NruI and Sal I digested the insert DNA of p7A6 (25kb) into 8 to 11 fragments. HaeIII, Sau 3A and XhoII generated numerous numbers of fragments. Sal I was selected for the first step of subcloning.

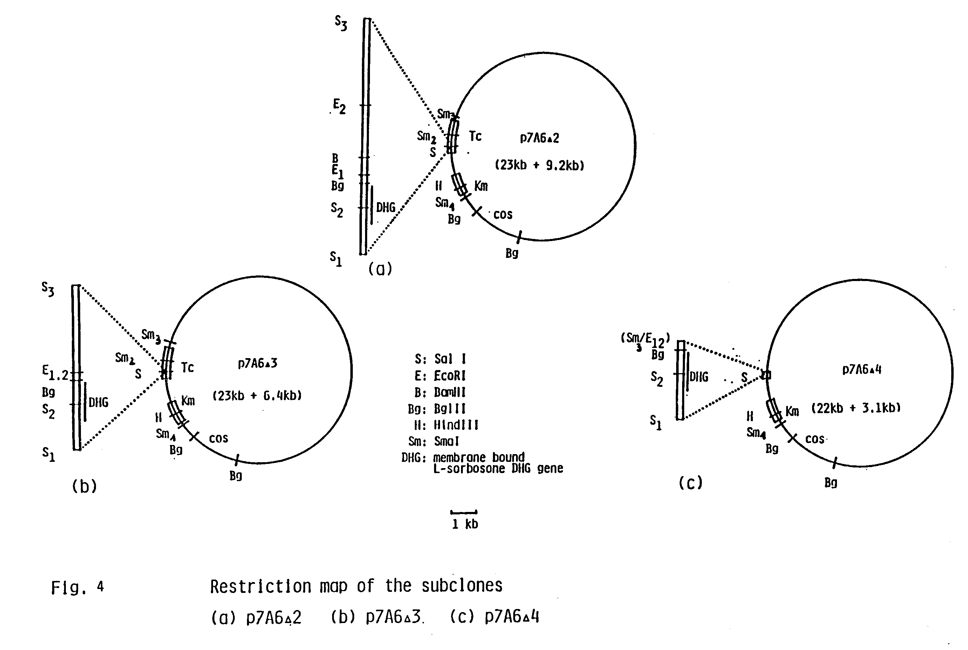

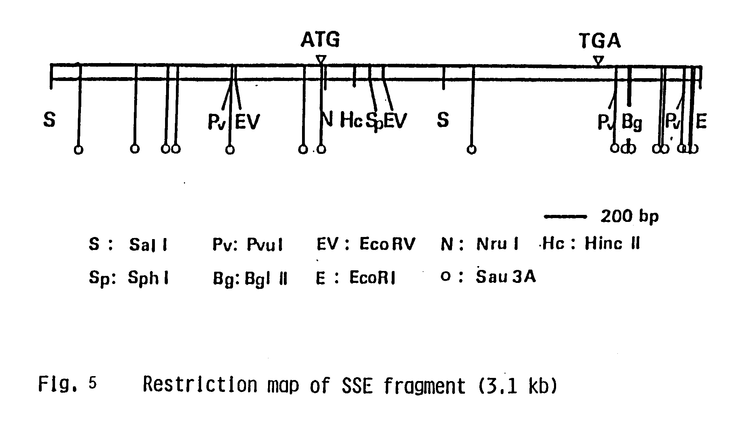

p7A6 was partially digested with Sal I, ligated with dephosphorylated pVK102 DNA digested with Sal I. The DNA mixture was transferred to E. coli S17-1 by transformation and then to G. oxydans OX-4 by bi-parental conjugation. The smallest subclone, p7A6$\Delta$2, was isolated from the mini-resting screening of 200 transconjugants and its size of the insert was 9.2kb (Fig. 4-(a)). Second subcloning was conducted by a deletion of the fragment $E_1$-$E_2$ of p7A6$\Delta$2. p7A6$\Delta$3 (Fig. 4-(b)) thus obtained was further shortened by deletion of the fragment $E_{1/2}$-$Sm_3$. The resulting subclone, p7A6$\Delta$4 (Fig. 4-(c)), contained a 3.1kb insert in pVK102 with a small deletion of $Sm_2$-$Sm_3$ in the vector DNA. The detailed restriction map of the 3.1kb $S_1$-$S_2$-$E_{1/2}$ (SSE) fragment is illustrated in Fig. 5.

**Claims**

1. A shuttle vector comprising one or more marker genes, two different replication origins one functional in Escherichia coli and one functional in Gluconobacter oxydans and a Mob site.

2. A vector as claimed in claim 1, wherein said marker genes are antibiotic resistance genes.

3. A vector as claimed in claim 1 or 2, which comprises one or more inserts.

4. A vector as claimed in claim 3, wherein said insert(s) are selected from the group consisting of DNA sequences having multicloning sites, expression control sequences, cos sites, terminator sequences, ribosome binding sites, DNA sequences encoding signal peptides and/or proteins.

5. A transconjugant of genus Gluconobacter or genus Acetobacter into which a vector of claims 1-4 is introduced.

6. A process for producing a transconjugant as claimed in claim 5, which comprises contacting a strain belonging to genus Gluconobacter or genus Acetobacter with a strain of Escherichia coli transformed with a vector defined in any one of claims 1-4, under a conjugal mating condition.

7. A process for producing a shuttle vector as claimed in claims 1-4, which comprises:
   (a) preparing a DNA containing marker genes;
   (b) preparing a DNA containing a replication origin functional in Escherichia coli;
   (c) preparing a DNA containing a replication origin functional in Gluconobacter;
   (d) preparing a DNA containing a Mob site; and
   (e) combining the DNAs described in (a) to (d) by digesting the said DNAs with an appropriate restriction enzyme and ligating them.

8. A process for producing a pro- or eukaryotic polypeptide, which process comprises introducing by transconjugation into a strain of genus Gluconobacter or genus Acetobacter a shuttle vector as claimed in claims 1-4 containing the DNA sequence coding for said polypeptide operatively linked with an expression control sequence, culturing the transconjugant under appropriate conditions of growth and isolating the desired polypeptide from the culture.

**Patentansprüche**

1. Ein Pendelvektor, der ein oder mehrere Markergene, zwei verschiedene Replikationsursprünge, wovon einer in Escherichia coli und einer in Gluconobacter oxydans funktionsfähig ist, und eine Mob-Stelle umfasst.

2. Ein wie in Anspruch 1 beanspruchter Vektor, worin besagte Markergene Antibiotikaresistenzgene sind.

3. Ein wie in Anspruch 1 oder 2 beanspruchter Vektor, der eine oder mehrere Einfügungen enthält.

4. Ein wie in Anspruch 3 beanspruchter Vektor, worin besagte Einfügungen aus der Gruppe bestehend aus DNS-Sequenzen mit Vielfachklonierungsstellen, Expressionskontrollsequenzen, cos-Stellen, Terminatorsequenzen, ribosomalen Bindungsstellen und DNS-Sequenzen, die für Signalpeptide und/oder Proteine kodieren, ausgewählt sind.

5. Eine Transkonjugante des Genus Gluconobacter oder des Genus Acetobacter, in die ein Vektor der Ansprüche 1-4 eingeführt ist.

**6.** Ein Verfahren zur Herstellung einer wie in Anspruch 5 beanspruchten Transkonjuganten, das das in Kontaktbringen eines zum Genus Gluconobacter oder zum Genus Acetobacter gehörenden Stammes mit einem mit einem in den Ansprüchen 1-4 definierten Vektor transformierten Stamm von Escherichia coli unter konjugalen Paarungsbedingungen umfasst.

**7.** Ein wie in den Ansprüchen 1-4 beanspruchtes Verfahren zur Herstellung eines Pendelvektors, das:
(a) die Herstellung von Markergenen enthaltender DNS;
(b) die Herstellung von einer einen in Escherichia coli funktionsfähigen Replikationsursprung enthaltenden DNS;
(c) die Herstellung von einer einen in Gluconobacter funktionsfähigen Replikationsursprung enthaltenden DNS;
(d) die Herstellung einer eine Mob-Stelle enthaltenden DNS; und
(e) die Zusammenfügung der in (a) bis (d) beschriebenen DNS durch Verdauen besagter DNS mit geeigneten Restriktionsenzymen und Verbinden umfasst.

**8.** Ein Verfahren zur Herstellung eines pro- oder eukaryotischen Polypeptids, welches Verfahren das Einführen durch Transkonjugation eines wie in den Ansprüchen 1-4 beanspruchten Pendelvektors, der die das besagte Polypeptid kodierende DNS-Sequenz wirksam mit einer Expressionskontrollsequenz verbunden enthält in einen Stamm des Genus Gluconobacter oder des Genus Acetobacter, die Kultivierung der Transkonjuganten unter geeigneten Wachstumsbedingungen und die Isolierung des gewünschten Polypeptids von der Kultur umfasst.

**Revendications**

**1.** Vecteur navette comprenant un ou plusieurs gènes marqueurs, deux origines de réplication, une fonctionnelle dans Escherichia coli et une fonctionnelle dans Gluconobacter oxydans, et un site Mob.

**2.** Vecteur selon la revendication 1, dans lequel lesdits gènes marqueurs sont des gènes de résistance à un antibiotique.

**3.** Vecteur selon la revendication 1 ou 2, comprenant un ou plusieurs segments d'insertion.

**4.** Vecteur selon la revendication 3, dans lequel le(s)dit(s) segment(s) d'insertion est(sont) choisi(s) parmi des séquences d'ADN comportant des sites de multiclonage, des séquences régulatrices d'expression, des sites COS, des séquences de terminateur, des sites de liaison ribosomique, des séquences d'ADN codant pour des peptides signal et/ou des protéines.

**5.** Transconjugant appartenant au genre Gluconobacter ou au genre Acetobacter, dans lequel est introduit un vecteur des revendications 1 à 4.

**6.** Procédé pour la production d'un transconjugant selon la revendication 5, comprenant la mise en contact d'une souche appartenant au genre Gluconobacter ou au genre Acetobacter avec une souche d'Escherichia coli transformée par un vecteur défini dans l'une quelconque des revendications 1 à 4, dans une condition d'appariement par conjugaison.

**7.** Procédé pour la production d'un vecteur navette selon les revendications 1 à 4, comprenant:
(a) la production d'un ADN contenant des gènes marqueurs;
(b) la production d'un ADN contenant une origine de réplication fonctionnelle dans Escherichia coli;
(c) la production d'un ADN contenant une origine de réplication fonctionnelle dans Gluconobacter;
(d) la production d'un ADN contenant un site Mob; et
(e) la combinaison des ADN décrits en (a) à (d), par digestion desdits ADN par une enzyme de restriction appropriée et ligature de ceux-ci.

**8.** Procédé pour la production d'un polypeptide de procaryote ou d'eucaryote, lequel procédé comprend l'introduction par transconjugaison, dans une souche appartenant au genre Gluconobacter ou au genre Acetobacter, d'un vecteur navette selon les revendications 1 à 4, contenant la séquence d'ADN codant pour ledit polypeptide, fonctionnellement liée à une séquence régulatrice d'expression, la culture du transconjugant dans des conditions convenables de croissance et l'isolement du polypeptide recherché à partir de la culture.

pSUP301 (5 Kb)
Km^r Ap^r

pGO3293S
9.9 Kb

Hinc II          Hinc II

LIGATION

pGE-1
11.9 Kb
Km^r

H:    Hind III
Hc:   Hinc II
P:    Pst I
Pv:   Pvu I
S:    Sal I
Sm:   Sma I
X:    Xho I

Fig. 1.

pGE 1

pGE -SNB 3

pGE -SNB 1

pGE -SNB 2

⊢⊣ ; 1 kb

P ; Pvu I

B ; Bgl II

→ ; sorbosone dehydrogenase gene

Fig. 2.

EP 0 381 027 B1

EP 0 381 027 B1

pGE 1
(11.9kb)

B  E   PPs    XP        Ps      P    B E  PPBB

H
Km$^r$ Mob

1kb

pGE-SNB2
(15.3kb)

B  E  PPs    XP        Ps     P   B      P        HP B

H
Km$^r$ Mob

P
SN DH

X
Km$^r$

B : Bgl ll     E : EcoRI    H : Hind lll

P : Pvu I     Ps : Pst I    X : Xho I

SN DH : membrane – bound    sorbosone dehydrogenase  gene

Km$^r$ : kanamycin resistance gene

Mob: Mob site for conjugal transfer

Fig. 3.

Fig. 4    Restriction map of the subclones

(a) p7A6Δ2    (b) p7A6Δ3.    (c) p7A6Δ4

S: Sal I
E: EcoRI
B: BamHI
Bg: BglII
II: HindIII
Sm: SmaI
DHG: membrane bound
L-sorbosone DHG gene

⊢ 1 kb

ATG

TGA

S          Pv EV        N Hc Sp EV      S                    Pv Bg    Pv  E

——— 200 bp

S : Sal I      Pv: Pvu I     EV : EcoRV    N : Nru I   ·Hc : Hinc II

Sp: Sph I      Bg: Bgl II    E : EcoRI     o : Sau 3A

Fig. 5      Restriction map of SSE fragment (3.1 kb)

EP 0 381 027 B1